# EUROPEAN PATENT APPLICATION

(11) **EP 3 231 404 A1**
(43) Date of publication of application: **18.10.2017**
(21) Application number: 17166659.7
(22) Date of filing: 13.04.2017
(51) Int. Cl.: A61F 13/14, A01K 13/00, A61F 15/00, A61F 13/06, A61F 13/12, A61D 9/02, A61F 13/00, D03D 15/00

(54) **PROTECTIVE SLEEVE**

(30) Priority: 13.04.2016 GB 201606367
(71) Applicant: KnitMesh Limited, Holywell, Flintshire CH8 9DP (GB)
(72) Inventor: Bingham, Lewis, Holywell, Flintshire CH8 9DP (GB)
(74) Representative: HGF Limited

(57) **Abstract**

A method of inhibiting access to a region of an appendage (2) of a being, the method includes surrounding the appendage (2) with a metallic mesh sleeve (1) and drawing an elongate element (10, 10') interlaced within the mesh thereby to reduce the size of a portion of the sleeve (1) to at least partially conform to the size and/or shape of the appendage (2).

## Description

This invention relates generally to protective sleeves. More specifically, although not exclusively, this invention relates to the use of protective sleeves to shield a wound, injury or other condition, particularly but not exclusively in animals and humans.

Animals are prone to injuries, particularly on limbs such as legs, paws and tails. Such injuries are normally treated by cleaning, disinfecting and in extreme cases suturing, but can also involve the application of topical treatments, such as medical ointments and the like. Following such treatment, it is common to cover the injury using some form of dressing. It is a known issue that animals will generally remove such dressings and may also contaminate the wound or remove the topical treatments by scratching, licking and so on.

Animals are also prone to developing skin conditions, for example resulting from an allergic reaction or even prolonged self-trauma or mutilation. One such conditions is lick granuloma, or acral lick dermatitis. This condition is normally caused or at least exacerbated by an urge to lick an intermediate or terminal portion of the leg, paw or tail, which can cause painful lesions.

Some known methods for preventing such behaviour involves the use of Elizabethan collars, battery-enhanced wraps, bandages or anti-licking ointments and strips. Each of these solutions have drawbacks that would be appreciated by the skilled person.

It is therefore a first non-exclusive object of the invention to provide a method and device which mitigates an animal's tendency to interfere with a wound or skin condition. It is a more specific non-exclusive object of the invention to provide such a method and device that at least mitigates the issues and drawbacks associated with known solutions. It is a yet further, more general non-exclusive object of the invention to provide an improved or alternative method and device.

Accordingly, a first aspect of the invention provides a method of inhibiting access to a region of a body part, e.g. an appendage of a being, the method comprising surrounding the body part or appendage with a mesh sleeve, e.g. a knitted mesh sleeve or metallic knitted mesh sleeve, shaped to at least partially conform to the size and/or shape of the body part or appendage.

As used herein, the term "being" includes humans and animals. It has been observed that the use of a metallic mesh sleeve inhibits access to the region it surrounds and provides a durable and conformable structure whilst allowing the area to breathe through the open structure of the mesh. The invention therefore provides an arrangement that is not easily removed and which prevents scratching or licking of areas prone to irritation (e.g. infected areas, dermatitis or other skin conditions) in animals and humans and can even prevent or at least inhibit self-harm.

The method may comprise reducing the size of a portion of the sleeve to at least partially conform to the size and/or shape of the body part or appendage. The method may comprise reducing the size of one or more portions of the sleeve which are adjacent a joint, for example at least one portion on each side of the joint. The reduction of the size of the portion may be effected or facilitated or carried out using an elongate element, which may surround or be received at least partially within the sleeve or within or interlaced or intermeshed with or within the mesh of the sleeve.

In embodiments, the method comprises drawing an elongate element interlaced within the mesh thereby to reduce the size of a portion of the sleeve to at least partially conform to the size and/or shape of the body part or appendage. The method may comprise drawing an elongate element, which may be interlaced within the mesh, to close the mesh, for example an end thereof. The method may comprise drawing the elongate element to close the mesh in order to form a sock or sock-like structure.

The method may comprise cutting and/or tying the elongate element, for example after it is drawn. In embodiments where the method comprises cutting and tying, the elongate element may be cut before or after being tied.

As used herein, the term "tying", "tie", "tied" and any associated term refers to portions of the elongate element being drawn together and fastened or secured, for example but not exclusively by twisting, forming a knot or bow or otherwise connecting or uniting the portions together, for example by a separate or integral fastening means or fastener.

Another aspect of the invention provides a mesh sleeve, e.g. a metallic mesh sleeve.

The sleeve may be specifically adapted for use in a method as described above.

An open end of the sleeve may be folded, rolled or beaded over itself, for example to at least partially conceal one or more mesh ends. The method may comprise folding, rolling or beading an open end of the sleeve, for example to at least partially conceal one or more mesh ends. The sleeve may comprise an elongate element interlaced within the mesh. The elongate element may be configured to be drawn, e.g. from the mesh, for example to reduce the size of a portion of the sleeve and/or to at least partially conform to the size and/or shape of the body part or appendage.

A further aspect of the invention provides a mesh sleeve, e.g. a knitted mesh sleeve or metallic knitted mesh sleeve, for inhibiting access to a region of a body part, e.g. an appendage of a being, the sleeve comprising an open end folded, rolled or beaded over itself to at least partially conceal one or more mesh ends and an elongate element interlaced within the mesh that is configured to be drawn to reduce the size of a portion of the sleeve to at least partially conform to the size and/or shape of the body part or appendage.

The sleeve may comprise two open ends.

The knitting may comprise warp knitting or weft knitting. We prefer that the knitting comprises weft knitting because the loops of the weft threads thereby extend substantially in a direction more suited to reducing the size of the sleeve via drawing of the elongate element.

Weft knitting comprises making an article by knitting means in which the loops made of each weft thread are formed substantially across the width of the article. Weft knitting is characterised by each weft thread being fed substantially at right angles to the direction in which the article is produced.

Warp knitting comprises making an article by knitting means in which the loops made of each warp thread are formed substantially along the length of the article. Warp knitting is characterised by each warp thread being fed substantially in line with the direction in which the article is produce.

Advantageously, knitting has been found to be beneficial because as the article is reduced in size to draw it about the appendage, the loops can be narrowed to accommodate the imposed restriction. This ensures that the overall thickness of the article, in the region of the reduced portion may not alter significantly. In contrast, an article fabricated from ring links can only reduce by stacking of successive links. Moreover, providing a knitted structure ensures that the article is conformable to the underlying appendage, even in the region of the reduced portion, which is not possible with ring links.

Another aspect of the invention provides a method of inhibiting access to a region of a body part, e.g. an appendage of a being, the method comprising surrounding the body part or appendage with a mesh sleeve, e.g. a metallic mesh sleeve, having two open ends and reducing the size of at least a portion of the sleeve to at least partially conform to the size and/or shape of the body part or appendage

A yet further aspect of the invention provides a mesh sleeve, e.g. a metallic mesh sleeve, for inhibiting access to a region of a body part, e.g. an appendage of a being, the sleeve comprising two open ends at least of which is folded, rolled or beaded over itself to at least partially conceal one or more mesh ends, wherein at least a portion of the sleeve is configured to be reduced in size to at least partially conform to the size and/or shape of the body part or appendage.

Each of the open ends may be folded, rolled or beaded over itself, for example to at least partially conceal one or more mesh ends. The method may comprise folding, rolling or beading each open end of the sleeve, for example to at least partially conceal one or more mesh ends.

The mesh sleeve may comprise a knitted mesh. The elongate element may be interlaced within the knit. At least one yarn of the knitted mesh may comprise a single, double or multiple filament. The elongate element may comprise a single, double or multiple filament. In some embodiments two or more or a plurality or all of the yarns, e.g. including the elongate element, may comprise double or multiple filaments and/or a plurality of yarns may comprise single filaments. The method may comprise drawing the or an elongate element interlaced within the mesh or a knit of the mesh to reduce the size or diameter of the portion of the sleeve.

The sleeve may be pre-formed or made from flat mesh and joined along its length. The sleeve is preferably sized and dimensioned to accommodate the body part or appendage to be surrounded, for example from a finger to a chest or abdominal region. The mesh or one or more holes or apertures therein or therethrough may be configured and/or sized and/or dimensioned to allow a medicament, such as a topical composition, to be introduced or applied therethrough. The sleeve may enable and/or the method may comprise the placement of a dressing, e.g. a wound dressing, beneath or under the sleeve, for example prior to its placement on the body part or appendage. The elongate element may be drawn to close the mesh, for example an end thereof. The sleeve may include a closed portion, e.g. a closed end, which may form a sock or sock-like structure.

The mesh may comprise interlocked asymmetrical loops of yarn, which may be moveable relative to one another. The method may comprise drawing the elongate element such that interlocked asymmetrical loops of yarn of the mesh move relative to one another. The elongate element may comprise asymmetrical loops interlaced with the yarns. The method may comprise drawing one of the loops, e.g. of the elongate element, such that interlocked asymmetrical loops of yarn of the mesh move relative to one another.

The elongate element may comprise a yarn of the knit, e.g. drawn to reduce the size of a portion, band, ring, section, segment or region of the sleeve, for example the portion, band, ring, section, segment or region of the sleeve through which the yarn extends. The method may comprise drawing a yarn of the knit to reduce the size of the portion of the sleeve through which the yarn extends. The method may comprise drawing a stitch, e.g. thereby to draw loops of the mesh into a waist.

Another aspect of the invention provides a method of inhibiting access to a region of a body part, e.g. an appendage of a being, the method comprising surrounding the body part or appendage with a knitted mesh sleeve, e.g. a knitted metallic mesh sleeve, and drawing a yarn of the knitted mesh thereby to reduce the size of a portion of the sleeve to at least partially conform to the size and/or shape of the body part or appendage.

The sleeve or at least one yarn thereof may comprise a corrosion resistant metal. The sleeve or at least one yarn thereof may comprise stainless steel. The sleeve or at least one yarn thereof may comprise an antibacterial or antimicrobial element. The sleeve or at least one yarn thereof may comprise silver or a silver alloy. The sleeve or at least one yarn thereof may comprise one or more or any combination of stainless steel, clad steel, galvanised steel, aluminium, tin or nickel plated copper, silver clad copper, phosphor bronze, brass, nickel, molybdenum, silver or silver alloy.

Another aspect of the invention provides the use of a mesh sleeve, e.g. a metallic mesh sleeve, to inhibit access to a lick granuloma lesion.

Another aspect of the invention provides the use of a mesh sleeve, e.g. a metallic mesh sleeve, to inhibit access to a region of a body part, e.g. an appendage, wherein the sleeve surrounds and at least partially conforms to the size and/or shape of the body part or appendage by an elongate element interlaced within the mesh drawn to reduce the size of a portion of the sleeve.

The elongate element may be tied. The mesh sleeve may comprises a knitted mesh, wherein the elongate element is interlaced within the knit. The mesh may comprise interlocked asymmetrical loops of yarn which are moveable relative to one another. The elongate element may comprise a yarn of the knit drawn to reduce the size of the portion of the sleeve through which the yarn extends. An open end of the sleeve may be folded, rolled or beaded over itself to at least partially conceal one or more mesh ends. The sleeve may comprises two open ends each of which is folded, rolled or beaded over itself to at least partially conceal one or more mesh ends.

The sleeve may comprise any one or more of the features described above. The yarn may be non-metallic, for example it may be plastic or polymeric or any other material that can be formed into a filament.

Another aspect of the invention provides the use of a mesh sleeve, e.g. a metallic mesh sleeve, to inhibit access to a region of a body part, e.g. an appendage, wherein the sleeve has two open ends and surrounds and at least partially conforms to the size and/or shape of the body part or appendage.

For the avoidance of doubt, any of the features described herein apply equally to any aspect of the invention. For example, the use or sleeve may comprise any one or more features of the method relevant to thereto and/or the method may comprise any one or more features or steps relevant to one or more features of the use or the sleeve.

Another aspect of the invention provides a computer program element comprising and/or describing and/or defining a three-dimensional design for use with a simulation means or a three-dimensional additive or subtractive manufacturing means or device, e.g. a three-dimensional printer or CNC machine, the three-dimensional design comprising an embodiment of the sleeve described above or any part thereof.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. For the avoidance of doubt, the terms "may", "and/or", "e.g.", "for example" and any similar term as used herein should be interpreted as non-limiting such that any feature so-described need not be present. Indeed, any combination of optional features is expressly envisaged without departing from the scope of the invention, whether or not these are expressly claimed. The applicant reserves the right to change any originally filed claim or file any new claim accordingly, including the right to amend any originally filed claim to depend from and/or incorporate any feature of any other claim although not originally claimed in that manner.

Embodiments of the invention will now be described by way of example only with reference to the accompanying drawings in which:
Figure 1 is a perspective view of a knitted metallic sleeve for use in a method according to an embodiment of the invention;
Figure 2 is a perspective view of an animal with a metallic sleeve fitted in accordance with a method of the invention to a hind leg;
Figure 3 is a perspective view of the sleeve of Figure 1 illustrating the drawing of yarns of the knit;
Figure 4 is a side view of a sleeve according to an embodiment of the invention;
Figure 5 is a perspective view of an animal with a metallic sleeve fitted in accordance with a method of the invention to a front leg; and
Figure 6 is a perspective view of an animal with metallic sleeves fitted in accordance with a method of the invention to a front paw and a tail.

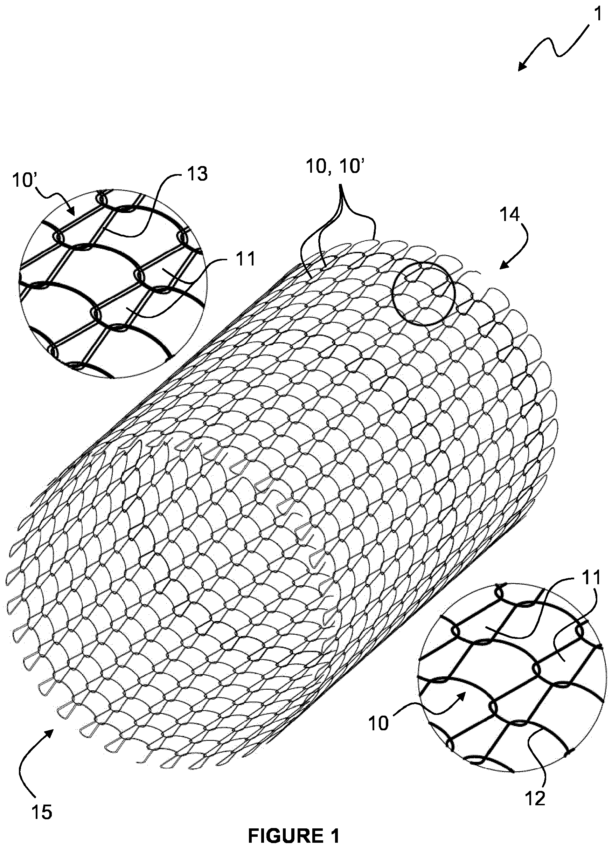

Referring now to Figure 1, there is shown a knitted mesh sleeve 1 formed of a plurality of metallic yarns 10, 10' interlaced to form interlocked asymmetrical loops 11 that are moveable relative to one another. The yarns 10, 10' may comprise single filaments 12 or multifilaments 13. In some embodiments, some of the yarns 10, 10' are single filaments 12 and others are multifilaments 13. The sleeve 1 also includes two open ends 14, 15 to form an open-ended tubular or cylindrical structure.

The sleeve 1 is knitted using a known method involving pulling the yarns from feeder spools, channeling them through a yarn feeder and knit by needles held in a cylinder head to form a tubular sleeve 1. It will be appreciated that the structure of the sleeve 1 may be varied, for example the size of the loops 11, the number of filaments 12, 13 forming the yarn or features of the filaments, such as gauge, material, coatings and the like. The configuration of the sleeve 1 may be altered by changing the number of feeder spools, yarn feeders and set-up, and size, arrangement and number of needles in the cylinder head, which enables various types of mesh to be knit.

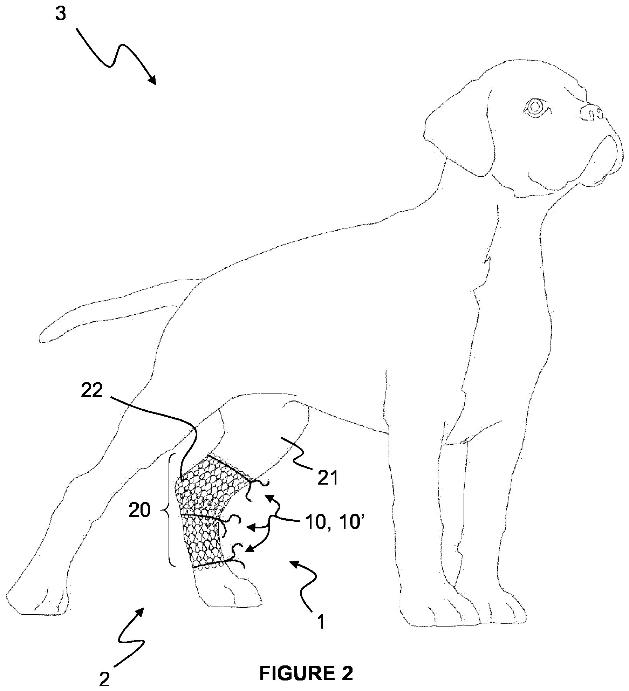

Referring now to Figure 2, in accordance with an embodiment of the invention the sleeve 1 is used to shield, protect and/or inhibit access to a region 20 of an appendage 2 of an animal 3, which is a hind leg 21 in this case. The region 20 may include an injury, wound, lesion, infected area or otherwise vulnerable part of the leg 21. The method involves placing the sleeve 1 over the leg 21 to surround the region 20 with and shaping the sleeve 1 to at least partially conform to the size and/or shape of the region 20 of the leg 21.

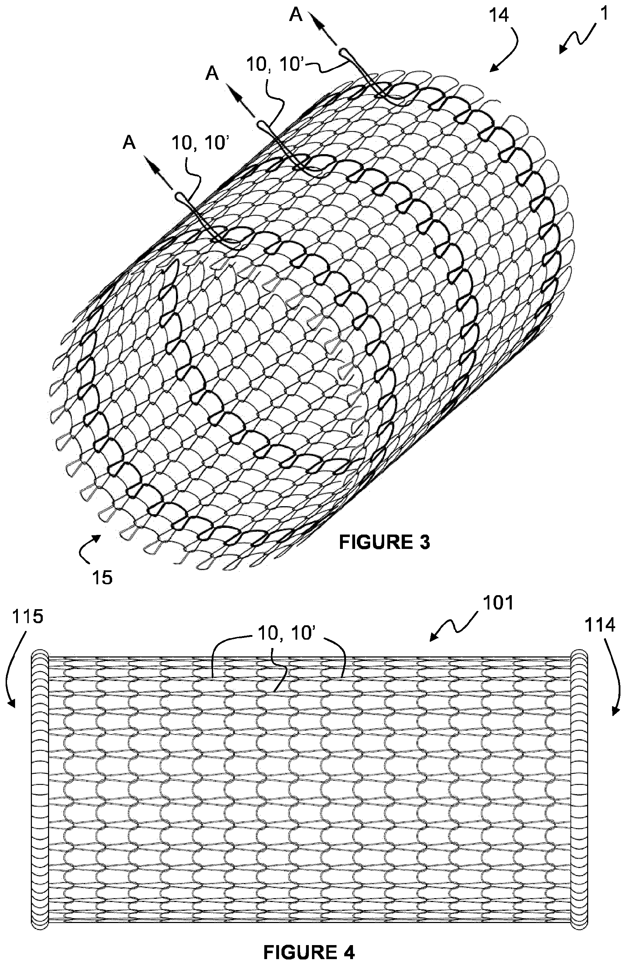

As shown more clearly in Figure 3, in this embodiment the size or diameter of the sleeve 1 is reduced by hooking one of the yarns 10, 10' and drawing it from the mesh thereby to reduce the size of a portion of the sleeve 1 through which the yarn 10, 10' extends. This enables the sleeve 1 to at least partially conform to the size and/or shape of the region 20 of the leg 21. The yarn 10, 10' is then tied to retain the reduced size of the sleeve portion, as shown in Figure 2. Optionally, stray portions 16 of the yarn 10, 10' may be cut and/or tucked into the mesh to prevent snagging.

It will be appreciated that the extent to which the yarn 10, 10' is drawn will depend upon the degree of reduction required to make safe the sleeve 1 without causing pain or injury to the animal 3. In some embodiments, the degree of reduction or constriction is limited to provide a loose fit, while preventing movement of the sleeve 1 along the appendage 2. For example, the method may involve hooking and drawing one or more yarns 10, 10' on either side of a joint 22 to reduce the size of respective portions on either side of the joint 22, thereby retaining it in the desired position.

In the case illustrated in Figure 2, three yarns 10, 10' were hooked and drawn, including one at each end 14, 15 of the sleeve 1 and a third immediately below the joint 22 of the hind leg 21. In other cases, a plurality of yarns 10, 10' may be hooked and drawn at regular intervals along the length of the sleeve 1 to bring the shape thereof into close conformity with the appendage 2.

Referring now to Figure 4, there is shown a sleeve 101 according to an embodiment of the invention similar to the sleeve 1 described above, wherein like references depict like features that will not be described further herein. The sleeve 101 in this embodiment differs from the embodiment described above in that each of the open ends 114, 115 is rolled over itself conceal the mesh ends.

It will be appreciated that this mitigates damage, injury or snagging of the mesh ends, thereby improving safety and comfort to the animal 3. It is also envisaged that the ends 114 may simply be folded over themselves or otherwise blunted or beaded.

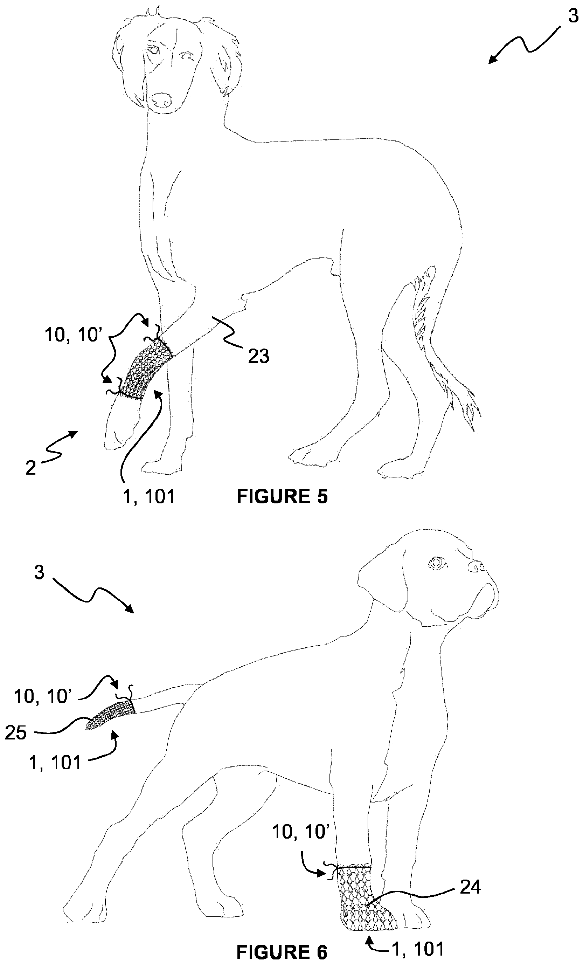

Figure 5 illustrates a sleeve 1,101 fitted to a front leg 23 of an animal 3. In this case, yarns 10, 10' adjacent each end 14, 15, 114, 115 of the sleeve 1, 101 were hooked and drawn.

As illustrated in Figure 6, one of the ends 14, 15, 114, 115 of the sleeve 1, 101 may be closed. This may be achieved, for example, by hooking one or more of the yarns 10, 10' at or adjacent the end to be closed and drawing it through to the extent that the end 14, 15, 114, 115 is effectively closed. In the case illustrated in Figure 6, one close-ended sleeve 1, 101 is fitted over a front paw 24 and another closed sleeve 1, 101 is fitted over the end of a tail 25.

In this embodiment, the sleeve 1, 101 is formed of a stainless steel. However, the sleeve 1, 101 or at least one yarn 10, 10' thereof may include any suitable material, preferably one that is corrosion resistant. The sleeve 1, 101 or at least one yarn 10, 10' thereof may advantageously include an antibacterial or antimicrobial element, either formed therein (e.g. an alloy or one or more inclusions) or thereon (e.g. a plating or coating). The sleeve 1, 101 or at least one yarn 10, 10' thereof may include silver or a silver alloy. The sleeve 1, 101 or at least one yarn 10, 10' thereof may include one or more or any combination of stainless steel, clad steel, galvanised steel, aluminium, tin or nickel plated copper, silver clad copper, phosphor bronze, brass, nickel, molybdenum, silver or silver alloy.

It will be appreciated by those skilled in the art that several variations to the aforementioned embodiments are envisaged without departing from the scope of the invention. For example, the yarn 10, 10' that is hooked and drawn in the arrangements described above may be omitted or replaced with any other elongate element. Whilst it is preferable that the elongate element 10, 10' is interlaced within the knit of the sleeve 1, 101, this is not strictly necessary in the case of some aspects of the invention. The yarns 10, 10' may be formed of more than two filaments 12, 13 and/or may be formed of any suitable material(s), provided they are consistent with the present disclosure. Whilst the mesh sleeve is preferably metallic, it may comprise any material that would discourage licking or nibbling.

It will also be appreciated by those skilled in the art that any number of combinations of the aforementioned features and/or those shown in the appended drawings provide clear advantages over the prior art and are therefore within the scope of the invention described herein.

The invention may be described by the following clauses:
1. A method of inhibiting access to a region of an appendage of a being, the method comprising surrounding the appendage with a metallic mesh sleeve and drawing an elongate element interlaced within the mesh thereby to reduce the size of a portion of the sleeve to at least partially conform to the size and/or shape of the appendage.
2. Method according to clause 1 comprising cutting the elongate element after it is drawn.
3. Method according to clause 1 or clause 2 comprising tying the elongate element after it is drawn.
4. Method according to any preceding clause, wherein the mesh sleeve comprises a knitted mesh, the elongate element being interlaced within the knit and drawn therefrom to reduce the size of the portion of the sleeve.
5. Method according to clause 2 comprising drawing the elongate element such that interlocked asymmetrical loops of yarn of the mesh move relative to one another.
6. Method according to clause 2 or clause 3, wherein the elongate element comprises a yarn of the knit that is drawn to reduce the size of the portion of the sleeve through which the yarn extends.
7. Method according to any preceding clause, wherein an open end of the sleeve is folded, rolled or beaded over itself to at least partially conceal one or more mesh ends.
8. Method according clause 7, wherein the sleeve comprises two open ends each of which is folded, rolled or beaded over itself to at least partially conceal one or more mesh ends.
9. Method according to any preceding clause, wherein the sleeve or at least one yarn thereof comprises a corrosion resistant metal.
10. Method according to any preceding clause, wherein the sleeve or at least one yarn thereof comprises stainless steel.
11. Method according to any preceding clause, wherein the sleeve or at least one yarn thereof comprises an antibacterial or antimicrobial element.
12. Method according to any preceding clause, wherein the sleeve or at least one yarn thereof comprises silver or a silver alloy.
13. A method of inhibiting access to a region of an appendage of a being, the method comprising surrounding the appendage with a metallic mesh sleeve having two open ends and reducing the size of at least a portion of the sleeve to at least partially conform to the size and/or shape of the appendage or to close an end of the sleeve.
14. Use of a metallic mesh sleeve to inhibit access to a lick granuloma lesion.
15. Use of a metallic mesh sleeve to inhibit access to a region of an appendage of a being, wherein the sleeve surrounds and at least partially conforms to the size and/or shape of the appendage by an elongate element interlaced within the mesh drawn to reduce the size of a portion of the sleeve.
16. Use according to clause 15, wherein the elongate element is tied.
17. Use according to clause 15 or clause 16, wherein the mesh sleeve comprises a knitted mesh, the elongate element being interlaced within the knit.
18. Use according to clause 17, wherein the mesh comprises interlocked asymmetrical loops of yarn which are moveable relative to one another.
19. Use according to clause 17 or clause 18, wherein the elongate element comprises a yarn of the knit drawn to reduce the size of the portion of the sleeve through which the yarn extends.
20. Use according to any one of clauses 15 to 19, wherein an open end of the sleeve is folded, rolled or beaded over itself to at least partially conceal one or more mesh ends.
21. Use according clause 20, wherein the sleeve comprises two open ends each of which is folded, rolled or beaded over itself to at least partially conceal one or more mesh ends.
22. Use according to any one of clauses 15 to 21, wherein the sleeve or at least one yarn thereof comprises a corrosion resistant metal.
23. Use according to any one of clauses 15 to 22, wherein the sleeve or at least one yarn thereof comprises stainless steel.
24. Use according to any one of clauses 15 to 23, wherein the sleeve or at least one yarn thereof comprises an antibacterial or antimicrobial element.
25. Use according to any one of clauses 15 to 24, wherein the sleeve or at least one yarn thereof comprises silver or a silver alloy.
26. Use of a metallic mesh sleeve to inhibit access to a region of an appendage of a being, wherein the sleeve has two open ends and surrounds and at least partially conforms to the size and/or shape of the appendage.
27. A metallic mesh sleeve specifically adapted for use in a method according to any one of clauses 1 to 13, wherein an open end of the sleeve is folded, rolled or beaded over itself to at least partially conceal one or more mesh ends.
28. A metallic mesh sleeve for inhibiting access to a region of an appendage of a being, the sleeve comprising an open end folded, rolled or beaded over itself to at least partially conceal one or more mesh ends and an elongate element interlaced within the mesh that is configured to be drawn to reduce the size of a portion of the sleeve to at least partially conform to the size and/or shape of the appendage.
29. Sleeve according clause 27 or clause 28, wherein the sleeve comprises two open ends each of which is folded, rolled or beaded over itself to at least partially conceal one or more mesh ends.
30. Sleeve according to any one of clauses 27 to 29, wherein the mesh sleeve comprises a knitted mesh, the elongate element being interlaced within the knit.
31. Sleeve according to any one of clauses 27 to 30, wherein the mesh comprises interlocked asymmetrical loops of yarn which are moveable relative to one another.
32. Sleeve according to any one of clauses 27 to 31, wherein the elongate element comprises a yarn of the knit drawn to reduce the size of the portion of the sleeve through which the yarn extends.
33. Sleeve according to any one of clauses 27 to 32, wherein the sleeve or at least one yarn thereof comprises a corrosion resistant metal.
34. Sleeve according to any one of clauses 27 to 33, wherein the sleeve or at least one yarn thereof comprises stainless steel.
35. Sleeve according to any one of clauses 27 to 34, wherein the sleeve or at least one yarn thereof comprises an antibacterial or antimicrobial element.
36. Sleeve according to any one of clauses 27 to 35, wherein the sleeve or at least one yarn thereof comprises silver or a silver alloy.
37. A metallic mesh sleeve for inhibiting access to a region of an appendage of a being, the sleeve comprising two open ends at least of which is folded, rolled or beaded over itself to at least partially conceal one or more mesh ends, wherein at least a portion of the sleeve is configured to be reduced in size to at least partially conform to the size and/or shape of the appendage or to close an end of the sleeve.
38. A method of inhibiting access to a region of an appendage substantially as described herein and/or as shown in the accompanying drawings.
39. Use of a metallic mesh sleeve to inhibit access to a region of an appendage substantially as described herein and/or as shown in the accompanying drawings.
40. A metallic mesh sleeve for inhibiting access to a region of an appendage substantially as described herein and/or as shown in the accompanying drawings.

## Claims

1. A method of inhibiting access to a region of an appendage of a being, the method comprising surrounding the appendage with a metallic knitted mesh sleeve and drawing an elongate element interlaced within the mesh thereby to reduce the size of a portion of the sleeve to at least partially conform to the size and/or shape of the appendage.

2. Method according to claim 1 comprising cutting the elongate element after it is drawn.

3. Method according to claim 1 or claim 2 comprising tying the elongate element after it is drawn.

4. Method according to any preceding claim, wherein the elongate element is interlaced within the knit and drawn therefrom to reduce the size of the portion of the sleeve.

5. Method according to claim 2 comprising drawing the elongate element such that interlocked asymmetrical loops of yarn of the mesh move relative to one another.

6. Method according to claim 2 or claim 3, wherein the elongate element comprises a yarn of the knit that is drawn to reduce the size of the portion of the sleeve through which the yarn extends.

7. Method according to any preceding claim, wherein an open end of the sleeve is folded, rolled or beaded over itself to at least partially conceal one or more mesh ends.

8. Method according claim 7, wherein the sleeve comprises two open ends each of which is folded, rolled or beaded over itself to at least partially conceal one or more mesh ends.

9. Method according to any preceding claim, wherein the sleeve or at least one yarn thereof comprises a corrosion resistant metal.

10. A metallic knitted mesh sleeve for inhibiting access to a region of an appendage of a being, the sleeve comprising an open end folded, rolled or beaded over itself to at least partially conceal one or more mesh ends and an elongate element interlaced within the mesh that is configured to be drawn to reduce the size of a portion of the sleeve to at least partially conform to the size and/or shape of the appendage.

11. Sleeve according claim 27 or claim 28, wherein the sleeve comprises two open ends each of which is folded, rolled or beaded over itself to at least partially conceal one or more mesh ends.

12. Sleeve according to any one of claims 27 to 29, wherein the mesh sleeve comprises a knitted mesh, the elongate element being interlaced within the knit.

13. Sleeve according to any one of claims 27 to 30, wherein the mesh comprises interlocked asymmetrical loops of yarn which are moveable relative to one another.

14. Sleeve according to any one of claims 27 to 31, wherein the elongate element comprises a yarn of the knit drawn to reduce the size of the portion of the sleeve through which the yarn extends.

15. Sleeve according to any one of claims 27 to 32, wherein the sleeve or at least one yarn thereof comprises a corrosion resistant metal.
